(19) Europäisches Patentamt
European Patent Office
Office européen des brevets

(11) Veröffentlichungsnummer: **0 286 970**
**A2**

(12) **EUROPÄISCHE PATENTANMELDUNG**

(21) Anmeldenummer: 88105502.4

(51) Int. Cl.⁴: **C07C 147/02 , A01N 47/06**

(22) Anmeldetag: 07.04.88

(30) Priorität: 14.04.87 DE 3712631

(43) Veröffentlichungstag der Anmeldung:
19.10.88 Patentblatt 88/42

(84) Benannte Vertragsstaaten:
AT BE CH DE FR GB IT LI NL

(71) Anmelder: **BAYER AG**
**Konzernverwaltung RP Patentabteilung**
**D-5090 Leverkusen 1 Bayerwerk(DE)**

(72) Erfinder: **Fest, Christa, Dr.**
**Im Johannistal 20**
**D-5600 Wuppertal 1(DE)**
Erfinder: **Hänssler, Gerd, Dr.**
**Am Arenzberg 58a**
**D-5090 Leverkusen 3(DE)**
Erfinder: **Brandes, Wilhelm, Dr.**
**Eichendorffstrasse 3**
**D-5653 Leichlingen(DE)**

(54) **Alpha-Methylsulfonyl-benzaldoxim-Derivate.**

(57) α-Methylsulfonyl-benzaldoxim-Derivate der allgemeinen Formel (I)

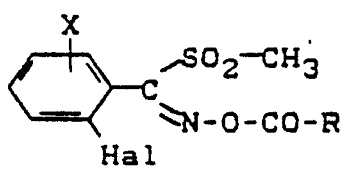

(I)

in welcher

R für Alkyl, Alkoxy, Halogenalkyl, Halogenalkoxy, Alkenyloxy, gegebenenfalls jeweils ein-bis mehrfach, gleich oder verschieden substituiertes Aryl oder Aryloxy, gegebenenfalls ein-bis mehrfach, gleich oder verschieden im Arylrest substituiertes Aralkyloxy, für gegebenenfalls ein-bis mehrfach, gleich oder verschieden substituiertes Cycloalkyloxy oder für einen gegebenenfalls ein-bis mehrfach, gleich oder verschieden substituierten Heterocyclus steht,

X für Wasserstoff oder Halogen steht und

Hal für Halogen steht, und ihre Verwendung zur Bekämpfung von Schädlingen.

Die neuen α-Methylsulfonyl-benzaldoxim-Derivate der Formel (I) können nach Analogieverfahren hergestellt werden, z.B. indem man geeignete α-Methylsulfonyl-benzaldoxime mit geeigneten Carbonylverbindungen umsetzt.

EP 0 286 970 A2

## α-Methylsulfonyl-benzaldoxim-Derivate

Die vorliegende Erfindung betrifft neue α-Methylsulfonyl-benzaldoxim-Derivate, ein Verfahren zu ihrer Herstellung und ihre Verwendung zur Bekämpfung von Schädlingen, insbesondere von Pilzen.

Es sind bereits eine Reihe von Aldoxim-Derivaten bekannt. So z.B. Arylsulfonylbenzaldoxime, wie das α-Phenylsulfonyl-2,6-dichlor-benzaldoxim, und ihre Verwendung als Schädlingsbekämpfungsmittel, vor allem ihre Verwendung in Mitteln zur Bekämpfung von Weizensteinbrand, sind bekannt (vgl. Schweizer Patent Nr. 423,350). Weiterhin sind α-(4-Methylphenylsulfonyl)-benzaldoxim-Derivate, wie z.B. das α-(4-Methylphenylsulfonyl)-2,6-dichlorbenzaldoxim-(4-chlor-3-methylphenyl)-carbamat, zur Bekämpfung von Schädlingen bekannt (vgl. DE-OS 3 520 943.

Es wurden neue α-Methylsulfonyl-benzaldoxim-Derivate der allgemeinen Formel (I)

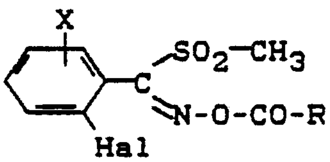

(I)

in welcher

R für Alkyl, Alkoxy, Halogenalkyl, Halogenalkoxy, Alkenyloxy, gegebenenfalls jeweils ein-bis mehrfach, gleich oder verschieden substituiertes Aryl oder Aryloxy, gegebenenfalls ein-bis mehrfach, gleich oder verschieden im Arylrest substituiertes Aralkyloxy, für gegebenenfalls ein-bis mehrfach, gleich oder verschieden substituiertes Cycloalkyloxy oder für einen gegebenenfalls ein-bis mehrfach, gleich oder verschieden substituierten Heterocyclus steht,

X für Wasserstoff oder Halogen steht und

Hal für Halogen steht,

gefunden.

Weiterhin wurde gefunden, daß man die α-Methylsulfonyl-benzaldoxim-Derivate der Formel (I)

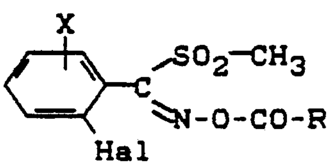

(I)

in welcher

R für Alkyl, Alkoxy, Halogenalkyl, Halogenalkoxy, Alkenyloxy, gegebenenfalls jeweils ein-bis mehrfach, gleich oder verschieden substituiertes Aryl oder Aryloxy, gegebenenfalls ein-bis mehrfach, gleich oder verschieden im Arylrest substituiertes Aralkyloxy, für gegebenenfalls ein-bis mehrfach, gleich oder verschieden substituiertes Cycloalkyloxy oder für einen gegebenenfalls ein-bis mehrfach, gleich oder verschieden substituierten Heterocyclus steht,

X für Wasserstoff oder Halogen steht und

Hal für Halogen steht,

erhält, wenn man

α-Methylsulfonyl-benzaldoxime der allgemeinen Formel (II)

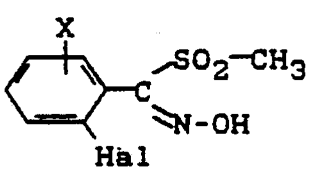

(II)

in welcher

X und Hal die oben angegebene Bedeutungen haben,

mit Carbonylverbindungen der allgemeinen Formel (III)

X - CO - R (III)

in welcher

R die oben angegebenen Bedeutungen hat und

X für ein Halogenatom, vorzugsweise Chlor, oder für den Rest -O-COR steht, worin R die oben angegebene Bedeutung hat,

gegebenenfalls in Gegenwart eines Lösungs-oder Verdünnungsmittels und gegebenenfalls in Gegenwart eines Säurebindemittels umsetzt.

Die erfindungsgemäßen α-Methylsulfonyl-benzaldoxim-Derivate der Formel (I) weisen starke biologische, vor allem fungizide Eigenschaften auf.

Überraschenderweise zeigen dabei die erfindungsgemäßen Verbindungen eine erheblich höhere vor allem fungizide Wirksamkeit als die aus dem Stand der Technik bekannten Verbindungen, die strukturell und wirkungsmäßig sehr naheliegende Verbindungen sind.

Die erfindungsgemäßen Verbindungen der Formel (I) können als syn-oder anti-Isomere oder als deren Gemische in unterschiedlicher Zusammensetzung anfallen. Die Erfindung bezieht sich sowohl auf die reinen Isomeren als auch auf die Isomerengemische.

Die Alkylreste in R sowie die Alkylteile in den Alkoxy-Resten in R können geradkettig oder verzweigt sein und enthalten vorzugsweise jeweils 1 bis 6, insbesondere 1 bis 4 Kohlenstoffatome. Beispielhaft seien genannt: Methyl, Ethyl, n-Propyl, iso-Propyl, n-Butyl, sec.-Butyl, iso-Butyl, tert.-Butyl, n-Pentyl, iso-Pentyl, sec.-Pentyl, n-Hexyl, sec.-Hexyl, Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, n-Hexoxy und sec.-Hexoxy.

Der Alkenyloxyrest in R enthält vorzugsweise 2 bis 6, insbesondere 2 bis 5, besonders bevorzugt 2 oder 3 Kohlenstoffatome. Beispielhaft seien genannt: Vinyl, Allyl, Propenyl(1), Butenyl und Pentenyl.

Die Halogenalkylteile in R in den Resten Halogenalkyl und Halogenalkoxy enthalten vorzugsweise jeweils 1 bis 6, insbesondere 1 bis 4, besonders bevorzugt 1 oder 2 Kohlenstoffatome und vorzugsweise 1 bis 9, insbesondere 1 bis 5, besonders bevorzugt 1 bis 4 gleiche oder verschiedene Halogenatome. Beispielhaft seien genannt: Monochlormethyl, Trichlormethyl, Trifluormethyl, Dichlorfluormethyl, Trichlorethyl, Tetrachlorethyl, Trichlormethoxy, Trichlorethoxy und Tetrachlorethoxy.

Halogen auch in den Resten wie Halogenalkyl, Halogenalkoxy oder in den Arylsubstituenten u.s.w. bedeutet Fluor, Chlor, Brom oder Iod, insbesondere Fluor oder Chlor, wenn an anderer Stelle nicht besonders definiert.

Aryl auch im Aryloxyrest in R kann für einen aromatischen Kohlenwasserstoffrest mit 6 bis 10 Kohlenstoffatomen stehen. Beispielsweise seien Phenyl und Naphthyl genannt. Bevorzugt ist Phenyl.

Aralkyl im Aralkoxy in R kann für einen Rest mit 7 bis 16 Kohlenstoffatomen stehen, wobei ein geradkettiger oder verzweigter Alkylrest ($C_1$ bis $C_6$) durch einen aromatischen Rest ($C_6$ bis $C_{10}$) substituiert sein kann. Beispielsweise seien Benzyl, Phenyl-ethyl und Phenyl-propyl genannt. Bevorzugt ist Benzyl.

Cycloalkyl im Cycloalkyloxy kann für einen cyclischen, bevorzugt monocyclischen Kohlenwasserstoffrest mit 5 bis 7 Kohlenstoffatomen stehen. Beispielsweise seien Cyclopentyl, Cyclohexyl und Cycloheptyl genannt. Bevorzugt sind Cyclopentyl und Cyclohexyl.

Heterocyclus kann für einen gesättigten, teilweise oder ganz ungesättigten 5-bis 8-gliedrigen Ring, vorzugsweise 5-oder 6-gliedrigen Ring mit 1 bis 3, vorzugsweise 1 oder 2, insbesondere 1 Heteroatom stehen, wobei als Heteroatome Schwefel, Sauerstoff oder Stickstoff, insbesondere Schwefel genannt seien.

Das genannte Aryl, Aryloxy und Aralkyloxy kann unsubstituiert oder substituiert sein; als Substituenten kommen 1 bis 5, bevorzugt 1 bis 3 Substituenten, besonders bevorzugt 1 oder 2 Substituenten aus der Gruppe der niederen Alkylgruppen mit 1 bis 4 Kohlenstoffatomen (Methyl, Ethyl, Propyl, Isopropyl, Butyl, Isobutyl), der niederen Alkoxygruppen mit 1 bis 4 Kohlenstoffatomen (Methoxy, Ethoxy, Propoxy, Isopropoxy, Butoxy, Isobutoxy) oder der Halogene (Fluor, Chlor, Brom) in Frage. Weitere Substituenten können sein: die Nitrogruppe sowie die Acetylgruppe.

Das genannte Cycloalkyloxy kann unsubstituiert oder substituiert sein; als Substituenten kommen 1 bis 5, bevorzugt 1 bis 3, besonders bevorzugt 1 oder 2 Substituenten aus der Gruppe der niederen Alkylgruppen mit 1 bis 4 Kohlenstoffatomen (Methyl, Ethyl, Propyl, i-Propyl, n-und s-Butyl, i-Butyl, t-Butyl) infrage.

Der genannte Heterocyclus kann unsubstituiert oder substituiert sein. Als Substituenten kommen 1 bis 3, bevorzugt 1 Substituent aus der Gruppe der niederen Alkyle mit 1 bis 4 Kohlenstoffatomen, wie oben aufgezählt, infrage.

Die erfindungsgemäßen α-Methylsulfonyl-bezaldoxim-Derivate sind durch die Formel (I) allgemein definiert. Bevorzugt sind Verbindungen der Formel (I),

in welcher

R für geradkettiges oder verzweigtes Alkyl mit 1 bis 6 Kohlenstoffatomen, für geradkettiges oder verzweigtes Alkoxy mit 1 bis 6 Kohlenstoffatomen, für Halogenalkyl oder Halogenalkoxy mit jeweils 1 bis 6 Kohlenstoffatomen im geradkettigen oder verzweigten Halogenalkylrest und 1 bis 9 gleichen oder verschiedenen Halogenatomen, für geradkettiges oder verzweigtes Alkenyloxy mit 2 bis 6 Kohlenstoffatomen, für unsubstituiertes oder jeweils ein-bis fünffach, gleich oder verschieden durch geradkettiges oder verzweigtes Alkyl mit 1 bis 4 Kohlenstoffatomen, geradkettiges oder verzweigtes Alkoxy mit 1 bis 4 Kohlenstoffatomen, Halogen, Nitro und Acetyl substituiertes Aryl oder Aryloxy mit 6 bis 10 Kohlenstoffatomen, für unsubstituiertes oder ein-bis fünffach, gleich oder verschieden durch geradkettiges oder verzweigtes Alkyl oder Alkoxy mit jeweils 1 bis 4 Kohlenstoffatomen, Halogen, Nitro und Acetyl substituiertes Aralkyloxy mit 6 bis 10 Kohlenstoffatomen im Arylrest und 1 bis 6 Kohlenstoffatomen im geradkettigen oder verzweigten Alkylrest, für unsubstituiertes oder ein-bis fünffach, gleich oder verschieden durch geradkettiges oder verzweigtes Alkyl mit 1 bis 4 Kohlenstoffatmen substituiertes Cycloalkyloxy mit 5 bis 7 Kohlenstoffatomen im Cycloalkylteil oder für einen unsubstituierten oder ein-bis dreifach, gleich oder verschieden durch geradkettiges oder verzweigtes Alkyl mit 1 bis 4 Kohlenstoffatomen substituierten, gesättigten, teilweise oder ganz ungesättigten Heterocyclus mit 5-bis 8-Ringgliedern steht, der 1 bis 3 Heteroatome enthalten kann;

X für Wasserstoff oder Halogen steht, und

Hal für Halogen steht.

Besonders bevorzugt sind Verbindungen der Formel (I),
in welcher

R für geradkettiges oder verzweigtes Alkyl mit 1 bis 4 Kohlenstoffatomen, für geradkettiges oder verzweigtes Alkoxy mit 1 bis 4 Kohlenstoffatomen, für Halogenalkyl oder Halogenalkoxy mit jeweils 1 bis 4 Kohlenstoffatomen im geradkettigen oder verzweigten Halogenalkrest und 1 bis 5 gleichen oder verschiedenen Halogenatomen, für geradkettiges oder verzweigtes Alkenyloxy mit 2 bis 5 Kohlenstoffatomen, für unsubstituiertes oder jeweils ein-bis fünffach, gleich oder verschieden durch geradkettiges oder verzweigtes Alkyl mit 1 bis 3 Kohlenstoffatomen, geradkettiges oder verzweigtes Alkoxy mit 1 bis 3 Kohlenstoffatomen, Halogen, Nitro und Acetyl substituiertes Phenyl oder Phenoxy, für unsubstituiertes oder ein-bis dreifach, gleich oder verschieden durch geradkettiges oder verzweigtes Alkyl oder Alkoxy mit jeweils 1 bis 3 Kohlenstoffatomen, Halogen, Nitro und Acetyl substituiertes Benzyloxy, Phenethyloxy oder Phenylpropyloxy, oder für unsubstituiertes oder ein-bis dreifach, gleich oder verschieden durch geradkettiges oder ver zweigtes Alkyl mit 1 bis 3 Kohlenstoffatomen substituiertes Cycloalkyloxy mit 5 bis 7 Kohlenstoffatomen im Cycloalkylteil oder für einen unsubstituierten oder einfach durch geradkettiges oder verzweigtes Alkyl mit 1 bis 3 Kohlenstoffatomen substituierten, gesättigten, teilweise gesättigten oder ungesättigten Heterocyclus mit 5 bis 7 Ringgliedern steht, der 1 oder 2 Schwefel-, Stickstoff-und/oder Sauerstoffatome enthalten kann;

X für Wasserstoff oder Halogen steht, und

Hal für Halogen steht.

Ganz besonders bevorzugt sind Verbindungen der Formel (I),
in welcher

R für geradkettiges oder verzweigtes Alkyl mit 1 bis 6 Kohlenstoffatomen, für geradkettiges oder verzweigtes Alkoxy mit 1 bis 6 Kohlenstoffatomen, für Halogenalkyl oder Halogenalkoxy mit jeweils 1 oder 2 Kohlenstoffatomen im geradkettigen oder verzweigten Halogenalkyl-bzw. -alkoxyrest und 1 bis 4 gleichen oder verschiedenen Halogenatomen, für geradkettiges oder verzweigtes Alkenyloxy mit 2 oder 3 Kohlenstoffatomen, für unsubstituiertes oder jeweils ein-oder zweifach, gleich oder verschieden durch Alkyl mit 1 oder 2 Kohlenstoffatomen, Alkoxy mit 1 oder 2 Kohlenstoffatomen, Halogen, Nitro und Acetyl substituiertes Phenyl oder Phenoxy, für unsubstituiertes oder ein-bis dreifach, gleich oder verschieden durch Alkyl oder Alkoxy mit jeweils 1 oder 2 Kohlenstoffatomen oder Halogen substituiertes Benzyl, für unsubstituiertes oder ein-bis dreifach, gleich oder verschieden durch Alkyl mit 1 oder 2 Kohlenstoffatomen substituiertes Cycloalkyloxy mit 5 oder 6 Kohlenstoffatomen im Cycloalkylteil oder für einen unsubstituierten oder ein-oder zweifach, gleich oder verschieden durch Alkyl mit 1 oder 2 Kohlenstoffatomen substituierten, ungesättigten Heterocyclus mit 5 oder 6 Ringgliedern steht, der Stickstoff-oder ein Schwefelatom enthalten kann;

X für Wasserstoff oder Halogen steht, und

Hal für Halogen steht.

Insbesondere seien Verbindungen der Formel (I) genannt,
in welcher

R für Methyl, Ethyl, Methoxy, Ethoxy, n-Propoxy, iso-Propoxy, iso-Butoxy, Allyloxy, 2,2,2-Trichlorethoxy, Benzyloxy, Chlormethyl, Phenyl, 2,6-Dichlorphenyl, 2,4-Dichlorphenyl, Phenoxy, Thienyl oder Cyclohexyloxy steht,

X für Wassertsoff, Fluor oder Chlor steht, und

Hal  für Fluor oder Chlor steht.

Verwendet man 2,6-Difluor-α-(4-methylsulfonyl)-benzaldoxim und Acetanhydrid als Ausgangsverbindungen, so kann der Reaktionsablauf des erfindungsgemäßen Verfahrens durch das folgende Reaktionsschema wiedergegeben werden:

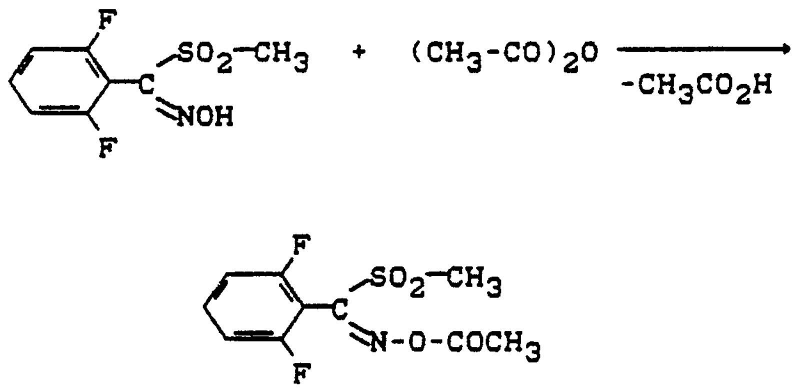


Die zur Durchführung des erfindungsgemäßen Verfahrens als Ausgangsstoffe benötigten α-Methylsulfonyl-benzaldoxime der allgemeinen Formel (II) sind neu. Die neuen Verbindungen können nach Analogieverfahren hergestellt werden, z.B. nach dem im Schweizer Patent Nr. 423.350 im Beispiel VII beschriebenen Verfahren, indem man α-Halogen-benzaldoxime der Formel (IV)

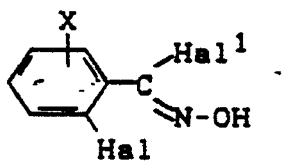

(IV)

worin

X und Hal die oben angegebene Bedeutung haben und

Hal[1] für Halogen, vorzugsweise für Chlor steht, mit Methansulfinsäuren der Formel (V)

$MSO_2-CH_3$

in welcher

M  für Wasserstoff oder ein Alkalimetalläquivalent steht,

gegebenenfalls in Gegenwart eines Lösungsmittels und gegebenenfalls in Gegenwart eines Säureakzeptors umsetzt.

Die Verbindungen der Formel (II) gehören nicht zum Stand der Technik und werden in einer nicht vorveröffentlichten Anmeldung der Anmelderin beschrieben.

Die Sulfinsäuren sind bekannte Verbindungen.

Die weiterhin als Ausgangsstoffe benötigten Carbonylverbindungen sind durch die Formel (III) definiert. Es handelt sich um bekannte Verbindungen der organischen Chemie.

Das erfindungsgemäße Verfahren kann gegebenenfalls in Gegenwart eines Lösungs-oder Verdünnungsmittels durch geführt werden. Als solche kommen prinzipiell alle inerten organischen Lösungsmittel infrage. Bevorzugt verwendet man Kohlenwasserstoffe, gegebenenfalls chlorierte, wie z.B. Benzol, Toluol, Xylol, Methylenchlorid, Chloroform, Tetrachlorkohlenstoff, Chlorbenzol und Dichlorbenzol, ferner Ether, wie Diethyl-und Dibutylether, Glykoldimethylether und Diglykoldimethylether, Tetrahydrofuran und Dioxan, weiterhin Ketone, wie Aceton, Methylethyl-, Methylisopropyl-und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid.

Als Säurebindemittel kommen für das erfindungsgemäße Verfahren übliche anorganische oder organische Säurebinder infrage. Als solche seien genannt: z.B. tert. Amine, wie Triethylamin, Pyridin, Triethylendiamin u.a.

Die Reaktionstemperatur des erfindungsgemäßen Verfahrens kann in einem größeren Temperaturbereich variiert werden. Im allgemeinen arbeitet man zwischen 0°C und 120°C, vorzugsweise zwischen 20°C und 70°C.

Die Reaktion wird normalerweise unter Normaldruck ausgeführt. Bei der Durchführung des erfindungsgemäßen Verfahrens legt man im allgemeinen die Verbindungen der Formel (II) in einem Lösungsmittel mit äquimolaren Mengen des Säurebinders vor und gibt die Carbonylverbindung der Formel (III) vorzugsweise ebenfalls in äquimolaren Mengen zu. Die Aufarbeitung erfolgt nach allgemein üblichen Methoden.

Eine besondere Ausführungsform ist noch zu erwähnen. Wenn X die Bedeutung -O-COR hat, d.h. also Carbonsäureanhydride der Formel (III) eingesetzt werden, arbeitet man ohne Lösungsmittel mit einem hohen Überschuß des Anhydrids, das dann gleichzeitig als Ausgangsstoff und Lösungsmittel dient. Die Aufarbeitung erfolgt ebenfalls nach üblichen Verfahren.

Die erfindungsgemäßen Wirkstoffe weisen eine starke biologische Wirkung auf und können zur Bekämpfung von unerwünschten Schädlingen praktisch eingesetzt werden. Die Wirkstoffe sind für den Gebrauch als Schädlingsbekämpfungsmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Phizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als

flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier-und/oder -schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die erfindungsgemäßen Verbindungen der Formel (I) zeigen eine besonders gute Wirkung gegen Erreger von Obstkrankheiten verursacht z.B. durch Venturia-Arten und Reiskrankheiten, verursacht durch Pyricularin-Arten ferner sei die gute Oomyceten-Wirkung und die in vitro-Wirkung gegen Bakterien erwähnt.

In entsprechenden Konzentrationen zeigen die erfindungsgemäßen Stoffe auch herbizide Wirkungen.

Anwendungsbeispiele:

In den folgenden Anwendungsbeispielen wurden die nachstehend aufgeführten Verbindungen als Vergleichssubstanzen eingesetzt:

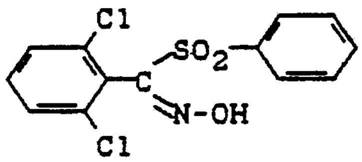 (A)

α-Benzsulfonyl-2,6-dichlorbenzaldoxin (bekannt aus CH 423.350; Beispiel VII),

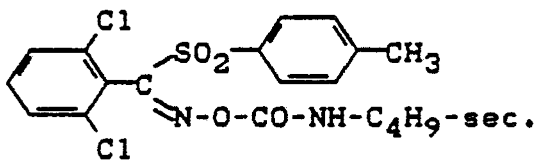 (B)

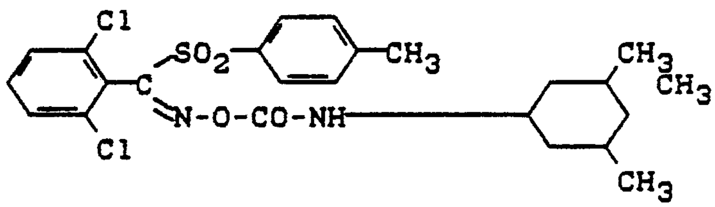 (C)

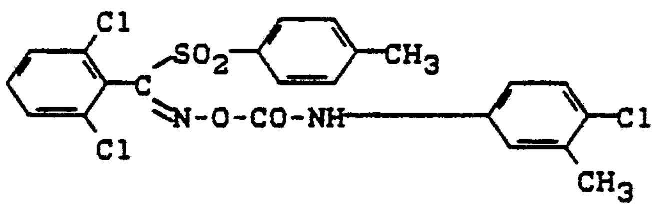 (D)

und

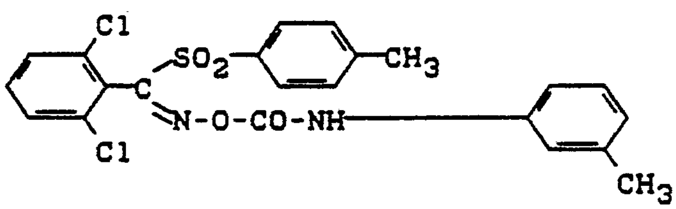 (E)

(B-E bekannt aus DE-OS 3 520 943).

Beispiel A

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen

Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksmkeit gegenüber dem Stand der Technik zeigen in diesem Test z.B. gegenüber die (A), (B) und (C) Verbindungen gemäß den Herstellungsbeispiele: 1, 4, 2 und 3.

Beispiel B

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. gegenüber (A), (D) und (E) die Verbindungen gemäß den Herstellungsbeispielen: 2 und 4.

Herstellungsbeispiele

Beispiel 1

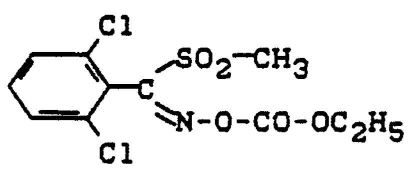

10 g (0,037 m) α-Methylsulfonyl-2,6-dichlorbenzaldoxim werden in 150 ml Acetonitril gelöst und mit 5,2 ml (o,037 m) Triethylamin versetzt. Bei Raumtemperatur werden 4 g (0,037 m) Chlorameisensäureethylester zugegeben. Die Reaktion verläuft schwach exotherm. Man hält das Reaktionsgemisch über Nacht bei Raumtemperatur, dann wird es auf ca. 750 ml Eiswasser gegossen, ausgerührt, abgesaugt, gewaschen und getrocknet. Anschließend wird mit Isopropanol verrührt, abgesaugt, mit Petrolether gewaschen und getrocknet.

Man erhält 10,6 g (84 % der Theorie) α-Methylsulfonyl-2,6-dichlorbenzaldoxim-O-ethylcarbonat vom Schmelzpunkt 169°C.

Analog werden die Verbindungen der Formel (I)

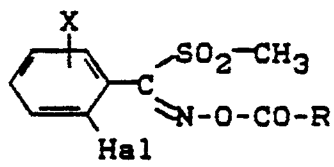

(I)

hergestellt:

| Beispiel-Nr. | X | Hal | R | Physikal. konstanten [Schmelzpunkt °C] |
|---|---|---|---|---|
| 2 | 2-Cl | Cl | $-OCH_2-CH=CH_2$ | 119° |
| 3 | 2-Cl | Cl | $-OCH_2-CCl_3$ | 153 |
| 4 | 2-F | F | $-OC_2H_5$ | 110 |
| 5 | 2-Cl | Cl | $-CH_3$ | 115 |
| 6 | 2-Cl | Cl | $-OCH_3$ | 170 |
| 7 | 2-Cl | Cl | $-OC_4H_9-i$ | 75 |
| 8 | 2-Cl | Cl | $-OCH_2-$ 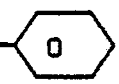 | 138 |
| 9 | 2-Cl | F | $-OCH_3$ | 154 |
| 10 | 2-Cl | F | $-OC_2H_5$ | 113 |
| 11 | 2-Cl | F | $-OCH_2-CH=CH_2$ | 76 |
| 12 | 2-Cl | F | $-OC_4H_9-i$ | 63 |
| 13 | 2-Cl | F | $-OCH_2CCl_3$ | 112 |
| 14 | 2-Cl | F | $-OCH_2-$ 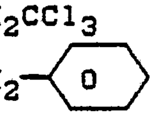 | 111 |

Herstellung der Vorprodukte

Beispiel 1A:

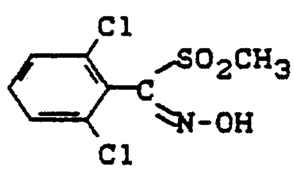

45 g (0,2 m) α-Chlor-2,6-dichlorbenzaldoxim werden in 200 ml Methanol gelöst und mit 22,5 g (0,2 m) Methansulfinsaurem-Natriumsalz versetzt. Die Reaktion verläuft exotherm. Das Reaktionsgemisch wird über Nacht bei Raumtemperatur gerührt. Anschließend wird es auf ca. 1 l Eiswasser gegossen, ausgerührt,

abgesaugt, gewaschen und getrocknet. Nach dem Umkristallisieren aus Isopropanol erhält man 35,5 g (66 % der Theorie) α-Methylsulfonyl-2,6-dichlorbenzaldoxim mit dem Schmelzpunkt 193°C.

Analog werden die Verbindungen der Formel (II)

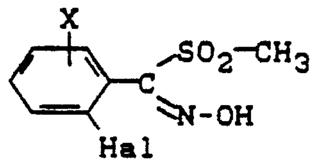

(II)

hergestellt:

| Beispiel-Nr. | X | Hal | Physikal. Konstanten [Schmelzpunkt °C] |
|---|---|---|---|
| 2A | H | Cl | 161 |
| 3A | 2-F | F | 153 |
| 4A | 2-Cl | F | 168 |

Das zur Herstellung von Beispiel 1A benötigte α-Chlor-2,6-dichlorbenzaldoxim kann z.B. analog den Angaben aus CH 423.350 (Beispiel II) hergestellt werden:

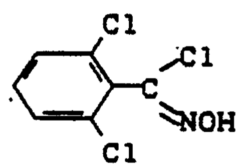

In eine Suspension von 19 Gew.-Teilen 2,6-Dichlorbenzaldoxim in 200 Volumenteilen Kohlenstofftetrachlorid wurde Chlor eingeleitet, bis nahezu alles Oxim in Lösung gegangen war. Die Temperatur der Reaktion wurde dabei unter 15°C gehalten. Die erhaltene grüne Lösung wurde evakuiert, um überschüssiges Chlor und eine flüchtige Substanz zu entfernen, vermutlich ein Nitrosylchorid. 21,2 Gew.-Teile Rückstand wurden teilweise beim Stehen zur Kristallisation gebracht. Der Rückstand wurde in 100 Volumen heißem 60/80° Petrolether gelöst. Beim Abkühlen erhielt man 10 Gew.-Teile Blättchen. F. 89-94°. Diese wurden abfiltriert und aus 60/80° Petrolether umkristallisiert und hatten dann einen Schmelzpunkt von 93,5 bis 94,5°.

Analyse:

Gefunden:

C 37,1 % H 1,9 %

$C_7H_4ONCl_3$ erfordert

C 37,4 % H 1,8 %

Aus der Mutterlauge wurde ein gelbes Öl isoliert und als 2,6-Dichlorbenzaldichlorid identifiziert.

**Ansprüche**

1. α-Methylsulfonyl-benzaldoxim-Derivate der allgemeinen Formel (I)

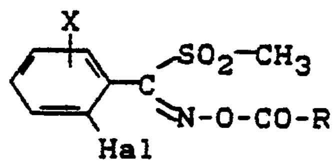

(I)

in welcher
R für Alkyl, Alkoxy, Halogenalkyl, Halogenalkoxy, Alkenyloxy, gegebenenfalls jeweils ein-bis mehrfach, gleich oder verschieden substituiertes Aryl oder Aryloxy, gegebenenfalls ein-bis mehrfach, gleich oder verschieden im Arylrest substituiertes Aralkyloxy, für gegebenenfalls ein-bis mehrfach, gleich oder verschieden substituiertes Cycloalkyloxy oder für einen gegebenenfalls ein-bis mehrfach, gleich oder verschieden substituierten Heterocyclus steht,
X für Wasserstoff oder Halogen steht und
Hal für Halogen setht,
deren Isomere und Isomerengemische.

2. α-Methylsulfonyl-benzaldoxim-Derivate gemäß Anspruch 1, worin in der Formel (I)
R für geradkettiges oder verzweigtes Alkyl mit 1 bis 6 Kohlenstoffatomen, für geradkettiges oder verzweigtes Alkoxy mit 1 bis 6 Kohlenstoffatomen, für Halogenalkyl oder Halogenalkoxy mit jeweils 1 bis 6 Kohlenstoffatomen im geradkettigen oder verzweigten Halogenalkylrest und 1 bis 9 gleichen oder verschiedenen Halogenatomen, für geradkettiges oder verzweigtes Alkenyloxy mit 2 bis 6 Kohlenstoffatomen, für unsubstituiertes oder jeweils ein-bis fünffach, gleich oder verschieden durch geradkettiges oder verzweigtes Alkyl mit 1 bis 4 Kohlenstoffatomen, geradkettiges oder verzweigtes Alkoxy mit 1 bis 4 Kohlenstoffatomen, Halogen, Nitro und Acetyl substituiertes Aryl oder Aryloxy mit 6 bis 10 Kohlenstoffatomen, für unsubstituiertes oder ein-bis fünffach, gleich oder verschieden durch geradkettiges oder verzweigtes Alkyl oder Alkoxy mit jeweils 1 bis 4 Kohlenstoffatomen, Halogen, Nitro und Acetyl substituiertes Aralkyloxy mit 6 bis 10 Kohlenstoffatomen im Arylrest und 1 bis 6 Kohlenstoffatomen im geradkettigen oder verzweigten Alkylrest, für unsubstituiertes oder ein-bis fünffach, gleich oder verschieden durch geradkettiges oder verzweigtes Alkyl mit 1 bis 4 Kohlenstoffatomen substituiertes Cycloalkyloxy mit 5 bis 7 Kohlenstoffatomen im Cycloalkylteil oder für einen unsubstituierten oder ein-bis dreifach, gleich oder verschieden durch geradkettiges oder verzweigtes Alkyl mit 1 bis 4 Kohlenstoffatomen substituierten, gesättigten, teilweise oder ganz ungesättigten Heterocyclus mit 5 bis 8 Ringgliedern steht, der 1 bis 3 Heteroatome enthalten kann;
X für Wasserstoff oder Halogen steht und
Hal für Halogen steht,
deren Isomeren und Isomerengemische.

3. α-Methylsulfonyl-benzaldoxim-Derivate gemäß Anspruch 1, worin in der Formel (I)
R für geradkettiges oder verzweigtes Alkyl mit 1 bis 4 Kohlenstoffatomen, für geradkettiges oder verzweigtes Alkoxy mit 1 bis 4 Kohlenstoffatomen, für Halogenalkyl oder Halogenalkoxy mit jeweils 1 bis 4 Kohlenstoffatomen im geradkettigen oder verzweigten Halogenalkylrest und 1 bis 5 gleichen oder verschiedenen Halogenatomen, für geradkettiges oder verzweigtes Alkenyloxy mit 2 bis 5 Kohlenstoffatomen, für unsubstituiertes oder jeweils ein-bis fünffach, gleich oder verschieden durch geradkettiges oder verzweigtes Alkyl mit 1 bis 3 Kohlenstoffatomen, geradkettiges oder verzweigtes Alkoxy mit 1 bis 3 Kohlenstoffatomen, Halogen, Nitro und Acetyl substituiertes Phenyl oder Phenoxy, für unsubstituiertes oder ein-bis dreifach, gleich oder verschieden durch geradkettiges oder verzweigtes Alkyl oder Alkoxy mit 1 bis 3 Kohlenstoffatomen, Halogen, Nitro und Acetyl substituiertes Benzyloxy, Phenethyloxy oder Phenylpropyloxy, und für unsubstituiertes oder ein-bis dreifach, gleich oder verschieden durch geradkettiges oder verzweigtes Alkyl mit 1 bis 3 Kohlenstoffatomen substituiertes Cycloalkyloxy mit 5 bis 7 Kohlenstoffatomen im Cycloalkylteil oder für einen unsubstituierten oder einfach durch geradkettiges oder verzweigtes Alkyl mit 1 bis 3 Kohlenstoffatomen substituierten, gesättigten, teilweise gesättigten oder ungesättigten Heterocyclus mit 5 bis 7 Ringgliedern steht, der 1 oder 2 Schwefel-, Stickstoff-und/oder Sauerstoffatome enthalten kann;
X für Wasserstoff oder Halogen steht und
Hal für Halogen steht,
deren Isomere und Isomerengemische.

4. α-Methylsulfonyl-benzaldoxim-Derivate gemäß Anspruch 1, worin in der Formel (I)

R für geradkettiges oder verzweigtes Alkyl mit 1 bis 6 Kohlenstoffatomen, für geradkettiges oder verzweigtes Alkoxy mit 1 bis 6 Kohlenstoffatomen, für Halogenalkyl oder Halogenalkoxy mit jeweils 1 oder 2 Kohlenstoffatomen im Halogenalkylrest und 1 bis 4 gleichen oder verschiedenen Halogenatomen, für geradkettiges oder verzweigtes Alkenyloxy mit 2 oder 3 Kohlenstoffatomen, für unsubstituiertes oder jeweils ein-oder zweifach, gleich oder verschieden durch Alkyl mit 1 oder 2 Kohlenstoffatomen, Alkoxy mit 1 oder 2 Kohlenstoffatomen, Halogen, Nitro und Acetyl substituiertes Phenyl oder Phenoxy, für unsubstituiertes oder ein-bis dreifach, gleich oder verschieden durch Alkyl oder Alkoxy mit 1 oder 2 Kohlenstoffatomen oder Halogen substituiertes Benzyl, für unsubstituiertes oder ein-bis dreifach, gleich oder verschieden durch Alkyl mit 1 oder 2 Kohlenstoffatomen substituiertes Cycloalkyloxy mit 5 oder 6 Kohlenstoffatomen im Cycloalkylteil oder für einen unsubstituierten oder ein-oder zweifach, gleich oder verschieden durch Alkyl mit 1 oder 2 Kohlenstoffatomen substituierten, ungesättigten Heterocyclus mit 5 oder 6 Ringgliedern steht, der Stickstoff-und/oder ein Schwefelatom enthalten kann;

X für Wasserstoff oder Halogen steht und

Hal für Halogen steht,

deren Isomere und Isomerengemische.

5. α-Methylsulfonyl-benzaldoxim-Derivate gemäß Anspruch 1, wobei in der Formel (I)

R für Methyl, Ethyl, Methoxy, Ethoxy, n-Propoxy, iso-Propoxy, iso-Butoxy, Allyloxy, 2,2,2-Trichlorethoxy, Benzyloxy, Chlormethyl, Phenyl, 2,6-Dichlorphenyl, 2,4-Dichlorphenyl, Phenoxy, Thienyl oder Cyclohexyloxy steht,

X für Wasserstoff, Fluor oder Chlor steht und

Hal für Fluor oder Chlor steht, deren Isomere und Isomerengemische.

6. Verfahren zur Herstellung von α-Methylsulfonylbenzaldoxim-Derivaten der Formel (I)

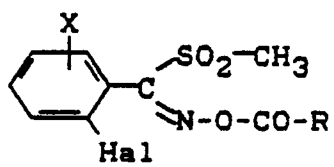

(I)

in welcher

R für Alkyl, Alkoxy, Halogenalkyl, Halogenalkoxy, Alkenyloxy, gegebenenfalls jeweils ein-bis mehrfach, gleich oder verschieden substituiertes Aryl oder Aryloxy, gegebenenfalls ein-bis mehrfach, gleich oder verschieden im Arylrest substituiertes Aralkyloxy, für gegebenenfalls ein-bis mehrfach, gleich oder verschieden substituiertes Cycloalkyloxy oder für einen gegebenenfalls ein-bis mehrfach, gleich oder verschieden substituierten Heterocyclus steht,

X für Wasserstoff oder Halogen steht und

$R^4$ für Halogen steht,

deren Isomere und Isomerengemische dadurch gekennzeichnet, daß man α-Methylsulfonyl-benzaldoxime der allgemeinen Formel (II)

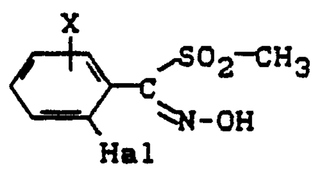

(II)

in welcher

X und Hal die oben angegebene Bedeutung haben,

mit Carbonylverbindungen der allgemeinen Formel (III)

X - CO - R (III)

in welcher

R die oben angegebenen Bedeutungen hat und

X für ein Halogenatom, oder für den Rest -O-COR steht, worin R die oben angegebene Bedeutung hat, gegebenenfalls in Gegenwart eines Lösungs-oder Verdünnungsmittels und gegebenenfalls in Gegenwart eines Säurebindemittels umsetzt.

7. Schädlingsbekämpfungsmittel, gekennzeichnet durch einen Gehalt an mindestens einem α-Methylsulfonyl-benzaldoxim-Derivat der Formel (I) nach den Ansprüchen 1 und 6.

8. Verwendung von α-Methylsulfonyl-benzaldoxim-Derivaten der Formel (I) nach den Ansprüchen 1 und 6 zur Bekämpfung von Schädlingen.

9. Verfahren zur Bekämpfung von Schädlingen, dadurch gekennzeichnet, daß man Benzaldoxim-Derivate der Formel (I) nach den Ansprüchen 1 und 6 auf Schädlinge und/oder ihren Lebensraum einwirken läßt.

10. Verfahren zur Herstellung von Schädlingsbekämpfungsmitteln, dadurch gekennzeichnet, daß man α-Methylsulfonyl-benzaldoxim-Derivate der Formel (I) nach den Ansprüchen 1 und 6 mit Streckmitteln und/oder oberflächenaktiven Mitteln vermischt.